# EUROPEAN PATENT SPECIFICATION

(11) **EP 4 368 120 B1**
(45) Date of publication and mention of the grant of the patent: **11.11.2026**
(21) Application number: 22855194.1
(22) Date of filing: 20.07.2022
(51) Int. Cl.: A61B 17/00, A61F 2/24, A61F 2/95

(54) **HANDLE, POSITIONING STRUCTURE, AND MEDICAL DEVICE**
GRIFF, POSITIONIERUNGSSTRUKTUR UND MEDIZINISCHE VORRICHTUNG
POIGNÉE, STRUCTURE DE POSITIONNEMENT ET DISPOSITIF MÉDICAL

(30) Priority: 09.08.2021 CN 202110908788
(43) Date of publication of application: 15.05.2024
(73) Proprietor: Shanghai Microport Cardioflow Medtech Co., Ltd., Shanghai 201203 (CN)
(72) Inventor: LIU, Shihong, Shanghai 201203 (CN); LIN, Xing, Shanghai 201203 (CN); JI, Lijun, Shanghai 201203 (CN); CHEN, Guoming, Shanghai 201203 (CN)
(74) Representative: Marks & Clerk LLP
(86) International application number: PCT/CN2022/106784
(87) International publication number: WO 2023/016211

(56) References cited:
- CN-A- 108 371 571
- CN-U- 209 984 374
- CN-U- 215 651 292
- CN-U- 215 651 293
- CN-U- 215 937 493
- US-A1- 2002 111 666
- US-A1- 2016 184 117
- US-A1- 2019 125 534
- US-A1- 2020 398 026
- US-A1- 2021 186 695

## Description

### TECHNICAL FIELD

The present invention relates to the technical field of medical instruments and, more specifically, to a handle, a positioning structure and a medical device.

### BACKGROUND

Interventional surgery is a brand-new treatment technology developed in recent years and is a minimally invasive surgical treatment. Interventional therapy is for introduction of special medical devices into the human body through a conveyor under the guidance of medical imaging equipment to diagnose and locally treat lesions in the body. Interventional treatment does not require surgery. Compared with traditional surgery, it has the advantages of less trauma, faster recovery, and better results. Interventional surgery can be used to treat heart valve disease or other diseases. The medical devices are different depending on the specific disease. For example, for heart valve disease, the medical device is an artificial valve, and for vascular disease, the medical device can be a medical stent.

The conveyor usually includes a handle and a catheter assembly. The catheter assembly is used to load medical devices and includes a movable tube. The handle is used to connect with the catheter assembly and control the movable tube to enable the medical device to be introduced into human body. As the power source during the entire interventional surgery, the handle needs to ensure sufficient safety, effectiveness and economy. There are different requirements for the operation of the handle at different stages of interventional surgery. Therefore, the handle needs to integrate multiple functions. In order to pursue a simplified structure of the handle, a certain part of the handle usually integrates multiple functions, resulting in complex assembly of the handle, and when one component fails, it may have an adverse impact on the functions of multiple other components directly connected to the component. In addition, the current handle structure requires high processing technology, which increases the difficulty and cost of processing. US2020398026A1 discloses a catheter including a handle with torquing and steering mechanisms. The torquing mechanism includes a rotatable nosecone and a bearing coupled to the nosecone to be rotatable therewith. The steering mechanism includes a rack coupled to the bearing to be slideable therewith and a pull wire having a proximal end attached to the bearing and a distal end attached to a distal portion of the shaft. US2002111666A1 discloses a medical device and procedure for accurately positioning a catheter across a desired region within a patient's vasculature. US2016184117A1 discloses a prosthesis delivery device and to a handle assembly at the distal end of the delivery device.

### SUMMARY OF THE INVENTION

An object of the present invention is to provide a handle, a positioning structure and a medical device. The positioning structure is assembled in the housing of the handle, which is conducive to simplifying the overall assembly process of the handle and improving the performance of the handle.

In a first aspect of the present invention there is provide a handle for interventional surgery according to claim 1. A second aspect of the present invention provides a medical device according to claim 14 which comprises a catheter assembly and a handle according to the first aspect of the present invention.

In order to achieve the above object, the present invention provides a handle, comprising:
a housing;
a positioning structure, at least partially disposed inside the housing, the positioning structure having a first inner cavity, the positioning structure comprising a positioning seat and a support arm, the positioning seat connected to the housing, the support arm connected to the positioning seat, the support arm being part of a side wall of the first inner cavity, and the support arm extending along an axial direction of the first inner cavity; and
a moving mechanism, at least partially inserted in the first inner cavity and configured to be movable along the axial direction of the first inner cavity.

Optionally, the support arm comprises at least two support sub-arms arranged at intervals along a circumference of the positioning seat, and an avoidance slot is formed between adjacent two of the support sub-arms;
wherein the handle further comprises a driving part connected to the housing; the moving mechanism comprises a moving rod and a transmission part provided on the moving rod, and the moving rod is at least partially inserted in the first inner cavity, the transmission part passes through the avoidance slot and is drivingly connected with the driving part.

Optionally, the at least two support sub-arms are arranged to be centrally or axially symmetrical along the circumference of the positioning seat.

Optionally, the driving part is provided with first transmission threads; a part of an outer surface of the moving rod is provided with second transmission threads that is formed as the transmission part and engaged with the first transmission threads.

Optionally, the housing comprises a main housing and a cover, and wherein a distal end of the driving part and a proximal end of the main housing are rotatably connected, and a proximal end of the driving part is rotatably connected to the cover.

Optionally, the positioning seat comprises a first positioning sub-seat and a second positioning sub-seat, and wherein the first positioning sub-seat is connected to a distal end of the support arm and to the main housing, and the second positioning sub-seat is connected to a proximal end of the support arm and to the cover.

Optionally, in a cross section of the first positioning sub-seat, the first inner cavity comprises a central area and an avoidance area, and wherein the central area is configured for allowing the moving rod to pass through, and the avoidance area is connected with the central area and is configured for the transmission part to pass through.

Optionally, in a radial direction of the positioning structure, an outer surface of the first positioning sub-seat protrudes from an outer surface of the support arm.

Optionally, the first positioning sub-seat is configured to remain circumferentially stationary relative to the main housing.

Optionally, the cover has a central through hole therein; the second positioning sub-seat comprises a first seat body and a second seat body, and wherein the first seat body is connected to the support arm, the second seat body is connected to a proximal end of the first seat body, and a cross-sectional area of the second seat body is smaller than an end face area of the proximal end of the first seat body; the second seat body passes through the central through hole.

Optionally, the second positioning sub-seat is configured to remain circumferentially stationary relative to the cover.

Optionally, a distal end of the first inner cavity is a first open end, and a proximal end of the first inner cavity is a second open end or a closed end.

Optionally, the positioning seat and/or the support arm are further defined with a weight-reducing structure.

In order to achieve the above object, the present invention also provides a positioning structure, wherein the positioning structure is the above positioning structure.

In order to achieve the above object, the present invention also provides a medical device, comprising a catheter assembly and the above handle, the catheter assembly comprising a movable tube, wherein the handle is disposed at a proximal end of the catheter assembly and is connected with the movable tube, and is configured to control the movable tube to move axially.

Optionally, the moving mechanism has a second inner cavity extending through the moving mechanism along an axial direction thereof; the catheter assembly comprises an inner tube and an outer tube sleeved outside the inner tube, and the outer tube is used as the movable tube, the outer tube is connected to a distal end of the moving mechanism, a proximal end of the inner tube extends out of the outer tube and extends through the second inner cavity.

Compared with the prior art, the handle, positioning structure and medical device of the present invention have the following advantages:
The handle includes a housing, a positioning structure and a moving mechanism. The positioning structure is at least partially disposed inside the housing, and the positioning structure has a first inner cavity. The positioning structure includes a positioning seat and a support arm. The positioning seat is connected to the housing. The support arm is connected to the positioning seat. The support arm may be a part of a side wall of the first inner cavity. The support arm extends along an axial direction of the first inner cavity. The moving mechanism is at least partially inserted in the first inner cavity and configured to be movable along the axial direction of the first inner cavity. That is to say, when the handle is being assembled, the moving mechanism is positioned and supported in the housing through the positioning structure, which simplifies the assembly of the components, reduces the interference between the components, and improves the safety for use and is also conducive to simplifying the overall assembly process of the handle and making it easier to process.

The support arm includes at least two support sub-arms arranged at intervals along a circumference of the positioning seat, and the at least two support sub-arms are preferably evenly arranged, and an avoidance slot is formed between adjacent two of the support sub-arms; the handle further comprises a driving part connected to the housing; the moving mechanism comprises a moving rod and a transmission part provided on the moving rod, and the moving rod is at least partially inserted in the first inner cavity, the transmission part passes through the avoidance slot and is drivingly connected with the driving part. In this way, in an aspect, it provides space for the assembly of the moving mechanism and the driving part, and an another aspect, the adjacent two support sub-arms can also provide circumferential and radial limits for the moving mechanism to prevent the moving mechanism from shaking or shifting, improve the stability of the transmission, and improve the performance of the handle.

### BRIEF DESCRIPTION OF THE DRAWINGS

The present invention will be better understood with reference to the accompanying drawings provided without limiting the invention, in which:
Fig. 1 is a schematic diagram of the overall appearance and structure of a handle according to an embodiment of the present invention;
Fig. 2 is a structural schematic diagram of a handle according to an embodiment of the present invention, in which the internal structure of the handle is shown;
Fig. 3 is a structural schematic diagram of a positioning structure of a handle according to an embodiment of the present invention;
Fig. 4 is a structural schematic diagram of a cover of a housing of a handle according to an embodiment of the present invention;
Fig. 5 is a structural schematic diagram of a positioning structure of a handle according to an alternative embodiment of the present invention, in which a first seat body and a second seat body are not formed on the second positioning sub-seat;
Fig. 6 is a schematic diagram showing an assembled structure of the positioning structure and the moving mechanism of the handle according to an embodiment of the present invention;
Fig. 7 is a schematic diagram showing an assembled structure of the positioning structure and the moving mechanism of the handle according to an embodiment of the present invention, which is viewed from a perspective different from that of Fig. 6;
Fig. 8 is a structural schematic diagram of the first positioning sub-seat of the positioning structure of the handle provided according to an embodiment of the present invention;
Fig. 9 is a structural schematic diagram of a positioning structure of a handle according to an alternative embodiment of the present invention, in which the distance from the outer peripheral surface of the first positioning sub-seat to the axis of the positioning structure is equal to the distance from the outer peripheral surface of the support arm to the axis of the positioning structure;
Fig. 10 is a structural schematic diagram of a medical device according to an embodiment of the present invention;

List of reference numerals:
10, positioning structure; 100, positioning seat; 101, first inner cavity; 101a, central area; 101b, avoidance area; 110, first positioning sub-seat; 111, first plane; 120, second positioning sub-seat; 121, first seat body; 122, second seat body; 123, step surface; 124, second plane; 200, support arm; 210, support sub-arm; 201, avoidance slot;
30, housing; 310, main housing; 320, cover; 321, central through hole; 322, second mounting groove; 323, limiting plane; 324, protruding portion;
40, moving mechanism; 410, moving rod; 420, transmission part; 430, transition tube; 440, second inner cavity;
50, driving part; 510, driving part body; 520, threaded sleeve; 521, first transmission threads;
60, catheter assembly; 610, inner tube; 620, outer tube.

### DETAILED DESCRIPTION

The following describes the embodiments of the present invention through specific examples. Those skilled in the art can easily understand other advantages and effects of the present invention from the content disclosed in this specification. The present invention can also be implemented or applied through other different specific embodiments. Various details in this specification can also be modified or changed in various ways based on different viewpoints and applications without departing from the scope of the present invention as defined in the appended claims. It should be noted that the diagrams provided in this embodiment only illustrate the basic concept of the present invention in a schematic manner. The drawings only show the components related to the present invention and do not follow the numbers, shapes and dimensions of components in actual implementation, the type, quantity and proportion of each component can be arbitrarily changed, and arrangement of component may also be more complex.

In addition, each embodiment described below has one or more technical features, but this does not mean that the inventor must implement all the technical features in any embodiment at the same time, or can only implement all or some of the technical features in different embodiments separately. In other words, on the premise that implementation is possible, those skilled in the art can, based on the disclosure of the present invention and design specifications or implementation requirements, selectively implement some or all of the technical features in any embodiment, or selectively implement a combination of some or all of the technical features in multiple embodiments, thereby increasing the flexibility of the implementation of the present invention.

As used in this specification, the singular forms "a", "an" and "the" include plural referents, and the plural form "plurality" includes two or more referents unless the content clearly dictates otherwise. As used in this specification, the term "or" is generally used in its sense including "and/or" unless the content clearly dictates otherwise, and the terms "mounted," "connected," and "connected" shall be understood in a broad sense, for example, it can be a fixed connection, a detachable connection, or an integral connection. The connection can be mechanical or electrical. The connection can also be achieved by one abuts against another. It can be a direct connection or an indirect connection through an intermediary. It can be an internal connection between two elements or an interaction between two elements. For those of ordinary skill in the art, the specific meanings of the above terms in the present invention can be understood according to specific circumstances.

The core idea of the present invention is to provide a handle, which includes a housing, a positioning structure and a moving mechanism. The positioning structure is at least partially disposed inside the housing, and the positioning structure has a first inner cavity. The positioning structure includes a positioning seat and a support arm. The positioning seat is connected to the housing. The support arm is connected to the positioning seat. The support arm is part of the side wall of the first inner cavity, and the support arm extends along the axial direction of the first inner cavity. The moving mechanism is at least partially inserted in the first inner cavity and is configured to be movable along the axis of the first inner cavity. When the handle is being assembled, the moving mechanism is positioned and supported in the housing through the positioning structure, which simplifies the assembly of the components, reduces the interference between the components, and improves the safety for use, and is also conducive to simplifying the overall assembly process of the handle and making it easier to process.

The handle is used in a medical device. The medical device includes the handle and a catheter assembly. The catheter assembly is used to load medical implants. The medical implants include but are not limited to medical stents, heart valve prostheses, embolization spring coils. The catheter assembly includes an inner tube and an outer tube that is sleeved outside the inner tube, and one of the inner tube and the outer tube serves as a movable tube. The handle is connected to the proximal end of the catheter assembly, and the moving mechanism is connected to the proximal end of the movable tube, so that the handle can control the movable tube to move axially so as to release the medical implant. For example, when the inner tube serves as the movable tube, the handle is configured to control the inner tube to move in the direction from proximal to distal, so that the medical implant can be released. When the outer tube serves as the movable tube, the handle is configured to control the outer tube to move in the direction from distal to proximal, so that the medical implant can be released.

It is to be noted that, as used herein, a "proximal end" and a "distal end" are relative orientations, relative positions and directions of elements or actions relative to each other from the perspective of a surgeon using the medical device. Although the "proximal end" and the "distal end" are not restrictive, the "proximal end" generally refers to the end of the medical device that is close to the surgeon during normal operation, while the "distal end" generally refers to the end that first enters the patient's body.

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

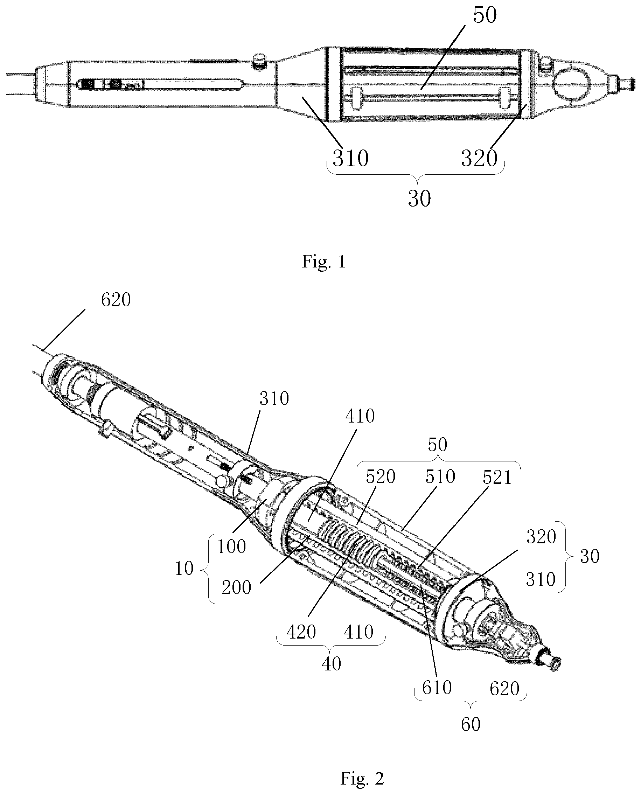

Fig. 1 is a structural schematic diagram of a handle according to an embodiment of the present invention; Fig. 2 is a schematic diagram showing the internal structure of the handle.

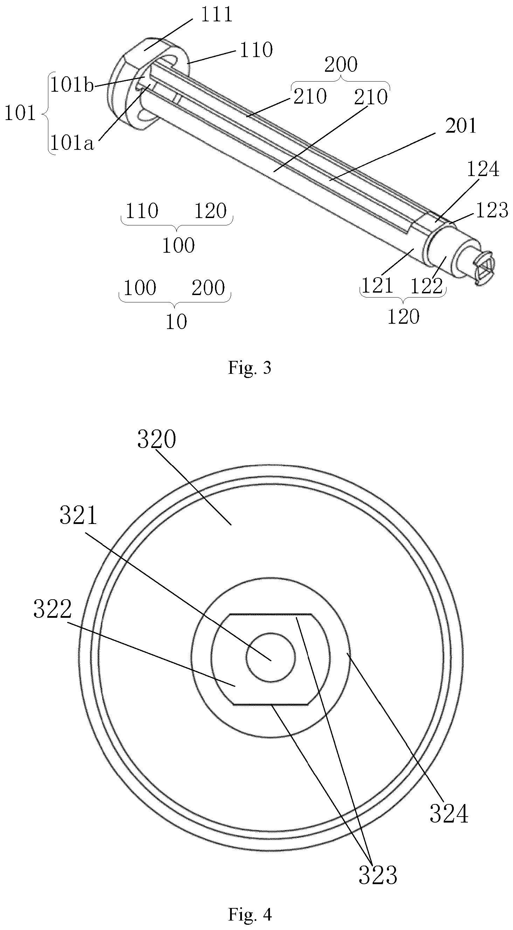

Please refer to Figs. 1 and 2, the handle includes a positioning structure 10, a housing 30 and a moving mechanism 40. Please refer to Fig. 2 in conjunction with Fig. 3, the positioning structure 10 is at least partially disposed inside the housing 30 and has a first inner cavity 101. The first inner cavity 101 extends in a first direction through the positioning structure 10. That is, the distal end and the proximal end of the first inner cavity 101 are both open, and respectively served as a first open end and a second open end. The first direction is, for example, the axial direction of the housing 30. Alternatively, the proximal end of the first inner cavity 101 may also be a closed end. The positioning structure 10 includes a positioning seat 100 and a support arm 200. The positioning seat 100 is connected to the housing 30. The support arm 200 is connected to the positioning seat 100. The support arm 200 is part of the side wall of the first inner cavity 101, and the support arm 200 extends along the axial direction of the first inner cavity 101. The axial direction of the first inner cavity 101 is the first direction. The moving mechanism 40 is at least partially inserted in the first inner cavity 101 and is configured to be movable along the axial direction of the first inner cavity 101. That is to say, when the handle is being assembled, the moving mechanism 40 is positioned and supported inside the housing 30 through the positioning structure 10, which simplifies the assembly of the positioning structure 10 and other structural components, reduces the interference between the components, and improves the safety for use, and is also conducive to simplifying the assembly process of the handle and making it easier to process.

In more detail, please refer to Fig. 3, the positioning seat 100 includes a first positioning sub-seat 110 and a second positioning sub-seat 120. The first positioning sub-seat 110 and the second positioning sub-seat 120 are respectively connected to the two ends of the support arm 200, and are connected to the housing 30 (the specific way of connection will be described later). Correspondingly, the first inner cavity 101 includes three parts that are connected with each other. One of the three parts is provided on the first positioning sub-seat 110, another one of the three parts extends through the support arm 200, and the rest of the three parts is provided on the second positioning sub-seat 120. The support arm 200 includes at least two support sub-arms 210 arranged at intervals along the circumference of the positioning seat 100, so that an avoidance slot 201 extending axially is formed between two adjacent support sub-arms 210 and is connected to the first inner cavity 101. The number of the avoidance slots 201 is the same as the number of the support sub-arms 210, that is, the number of the avoidance slots 201 is at least two.

In the embodiment of the present invention, it is preferable that the positioning seat 100 (including the first positioning sub-seat 110 and the second positioning sub-seat 120) and the support arm 200 (including all the support sub-arms 210) are integrally formed, to improve the overall rigidity of the positioning structure. Materials for the positioning structure 10 may include polymer materials or metal materials. The polymer materials may include but are not limited to at least one of ABS resin (acrylonitrile-butadiene-styrene copolymer), PC (polycarbonate), POM (polyoxymethylene resin), PEI (polyetherimide), PSU (polysulfone), PPSU (polyphenylsulfone resin), and PEEK (polyetheretherketone). The metal materials may include but are not limited to iron, chromium, manganese and their alloys. In addition, a weight-reducing structure (not shown in the figures) may also be designed on the positioning seat 100 and/or the support arm 200 to reduce the weight of the positioning structure 10 and thus the weight of the handle. The weight-reducing structure is, for example, a hole or other hollow structure.

Please refer to Fig. 2 in conjunction with Fig. 4, the housing 30 includes a main housing 310 and a cover 320, and the cover 320 may be disposed at the proximal end of the main housing 310. The inner wall of the main housing 310 defines thereon a first mounting groove (not shown in the figures), the cover 320 defines therein a central through hole 321, and the distal end face of the cover 320 defines thereon a second mounting groove 322. The first positioning sub-seat 110 is connected to the distal end of the support arm 200 and is mounted in the first mounting groove. The second positioning sub-seat 120 includes a first seat body 121 and a second seat body 122. The first seat body 121 is connected to the proximal end of the support arm 200, and the second seat body 122 is connected to the proximal end of the first seat body 121, and the cross-sectional area of the second seat body 122 is smaller than the area of the proximal end face of the first seat body 121, so that part of the proximal end face of the first seat body 121 is not covered by the second seat body 122 to form a step surface 123. The second seat body 122 passes through the central through hole 321 of the cover 320, so that the first seat body 121 is at least partially inserted into the second mounting groove 322. In this case, the distal end face of the cover 320 is pressed against the step surface 123.

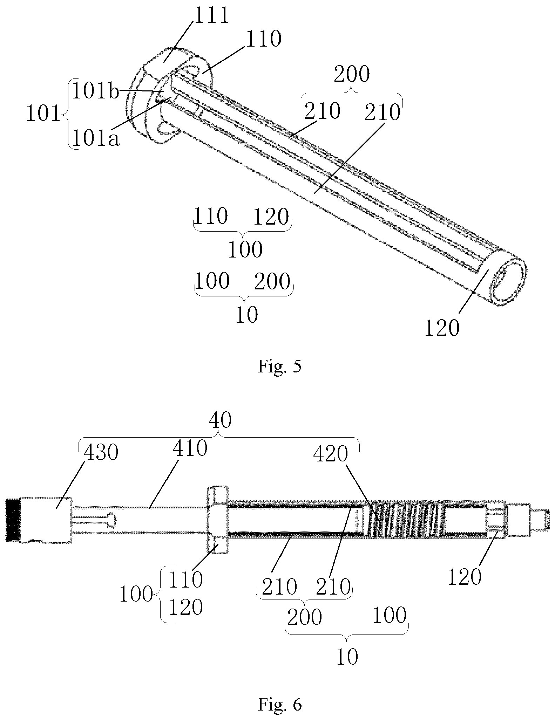

Furthermore, a fixing member can be used to remain the cover 320 and the second positioning sub-seat 120 to be stationary relative to each other in the axial direction to prevent the cover 320 from falling off from the proximal end of the second seat body 122. The fixing member is, for example, a nut (in this case, matched threads are formed on the second seat body 122). In addition, it can also be understood that the second positioning sub-seat 120 may not be divided into a first seat body and a second seat body (as shown in Fig. 5). In this case, the second positioning sub-seat 120 may be connected to the cover 320 in other ways. For example, the second positioning sub-seat 120 is at least partially inserted into the second mounting groove 322 and is glued to the side wall of the second mounting groove 322. In addition, a protruding portion 324 may be provided on the distal end face of the cover 320, and the second mounting groove 322 may be defined on the protruding portion 324.

Preferably, the first positioning sub-seat 110 is further configured to remain circumferentially stationary relative to the main housing 310. For this purpose, as shown in Figs. 3 and 5, the first positioning sub-seat 110 may have a non-rotational shape, that is, the cross-section of the first positioning sub-seat 110 is a non-circular shape. For example, a part of the outer peripheral surface of the first positioning sub-seat 110 is formed into a first plane 111. The shape and size of the cross section of the first mounting groove match with the shape and size of the cross section of the first positioning sub-seat 110. The outer peripheral surface of the first positioning sub-seat 110 refers to the outer surface of the first positioning sub-seat 110 that is parallel to the axis of the positioning seat 100.

The second positioning sub-seat 120 is configured to remain circumferentially stationary relative to the cover 320. In one implementation, as shown in Fig. 4, the cross section of the second mounting groove 322 has a non-circular shape. For example, part of the side wall of the second mounting groove 322 is formed as a limiting plane 323. As shown in Fig. 3, the first seat body 121 is at least partially inserted into the second mounting groove 322, and the shape and size of the cross section of the part of the first seat body 121 inserted into the second mounting groove 322 match with the shape and size of the cross-section of the second mounting groove 322. That is to say, a part of the outer peripheral surface of the first seat body 121 is formed into a second plane 124 that matches with the limiting plane 323. The outer peripheral surface of the first seat body 121 refers to the outer surface of the first seat body 121 that is parallel to the axis of the positioning seat 100.

Please refer back to Figs. 1 and 2, the handle also includes a driving part 50 that is provided on the housing 30 and is configured to drive the moving mechanism 40 to move along the axial direction of the housing 30.

Optionally, please continue to refer to Figs. 1 and 2, the driving part 50 is a manual driving mechanism and is rotatably connected to the housing 30. In detail, the main housing 310 and the cover 320 are not directly connected. The driving part 50 covers the support arm 200, and the distal end of the driving part 50 is rotatably connected to the proximal end of the main housing 310, and the proximal end of the driving part 50 is rotatably connected to the distal end of the cover 320.

The moving mechanism 40 includes a moving rod 410 and a transmission part 420 provided on the moving rod 410. The moving rod 410 is at least partially inserted in the first inner cavity 101. The transmission part 420 passes through the avoidance slot 201 between two adjacent support sub-arms 210 and is connected to the driving part 50 so that the driving part 50 can transmit driving force through the transmission part 420, thereby driving the moving mechanism 40 to move axially relative to the positioning structure 10.

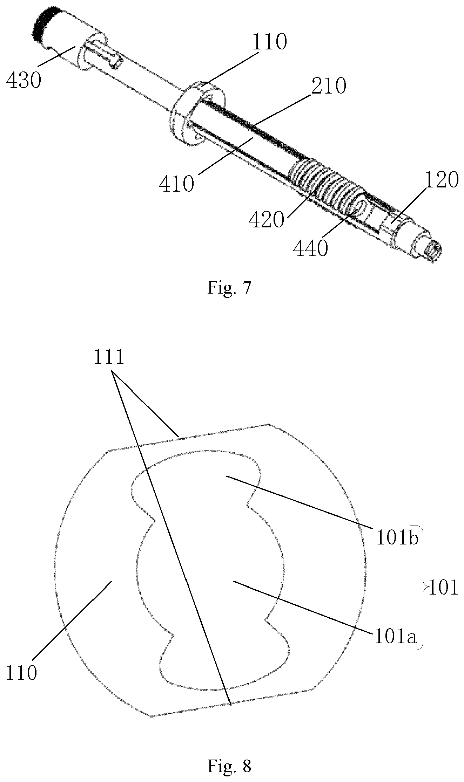

Please continue to refer to Fig. 2, combined with Figs. 6 and 7, the driving part 50 is provided with first transmission threads 521. Specifically, the driving part 50 preferably includes a driving part body 510 and a threaded sleeve 520 connected to the driving part body 510. The first transmission threads 521 are provided on the inner surface of the threaded sleeve 520. The outer surface of the moving rod 410 of the moving mechanism 40 is provided with second transmission threads engaging with the first transmission threads 521 for spiral transmission. That is to say, the second transmission threads form the transmission part 420, and the avoidance slot 201 between two adjacent support sub-arms 210 provides a space for the connection between the driving part 50 and the transmission part 420, and the avoidance slot 201 also limits the moving mechanism 40 in the circumferential and radial directions to prevent the moving mechanism 40 from shaking or shifting, so that the moving mechanism 40 can move stably along the axial direction. It can be understood that, since the second transmission threads needs to pass through the avoidance slot 201, the second transmission threads do not extend continuously in the circumferential direction of the moving rod, but includes a plurality of threaded segments arranged circumferentially at intervals, and each of the threaded segments passes through a corresponding one of the avoidance slots 201.

It is further preferred that the at least two support sub-arms 210 are arranged to be centrally or axially symmetrical along the circumference of the positioning seat 100, preferably, centrally symmetrical about any point on the axis of the positioning seat 100, or axially symmetrical about the axis of the positioning seat 100, and each avoidance slot 201 is configured for allowing a threaded segment of the second transmission threads to pass through. In this way, the force on the positioning structure 10 can be improved to be evenly distributed.

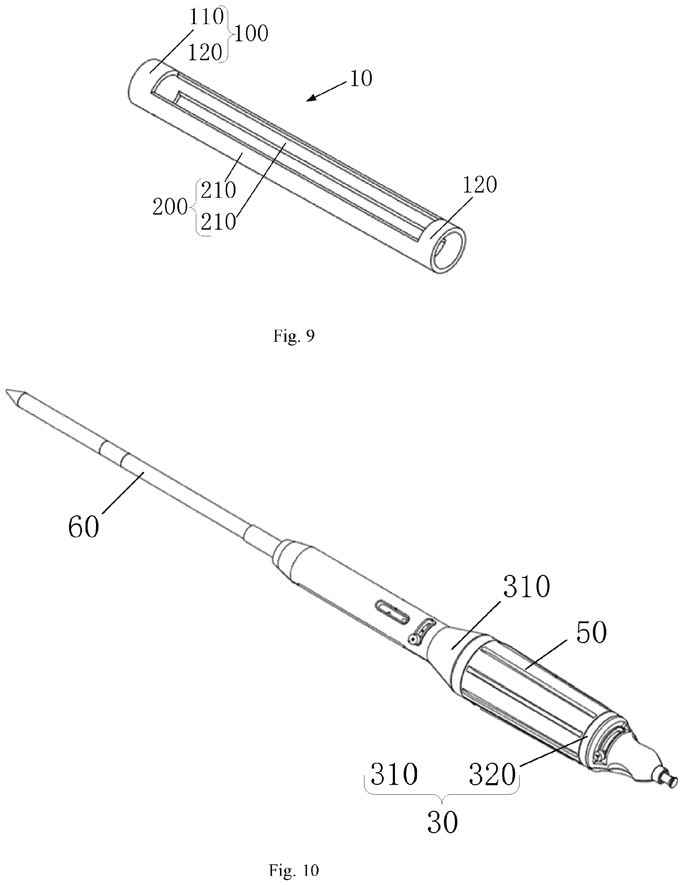

Further preferably, as shown in Figs 3, 5 and 8, in the cross section of the first positioning sub-seat 110, the first inner cavity 101 includes a central area 101a and at least one avoidance area 101b. The at least one avoidance area 101b is connected with the central area 101a, and in the circumferential direction of the first positioning sub-seat 110, each avoidance area 101b is located between two adjacent support sub-arms 210, so that the avoidance area 101b is arranged in correspondence with the avoidance slot 201. The shape and size of the central area 101a match with the shape and size of the cross section of the moving rod 410 to allow the moving rod 410 to pass through. The shape and size of each avoidance area 101b match with the shape and size of the threaded segment of the second transmission threads to allow the threaded segment to pass through. The purpose of this is to facilitate the assembly of the positioning structure and the moving mechanism 40, and to further limit the moving mechanism 40 in the circumferential and radial directions. In an alternative embodiment, the cross section of the first inner cavity on the first positioning sub-seat 110 is not divided into a central area and an avoidance area, but is in the form of a circle (as shown in Fig. 9) for allowing the moving rod 410 to pass through.

Please refer back to Fig. 2 in combination with Figs. 3 and 5, in this embodiment, the maximum inner diameter of the housing 30 may be equal to the inner diameter of the driving part body 510. Here, the distance from the outer peripheral surface of the first positioning sub-seat 110 to the axis of the positioning seat 100 should be greater than the distance from the outer peripheral surface of the support sub-arm 210 to the axis of the positioning seat 100, that is, in the radial direction of the positioning structure 10, the first positioning sub-seat 110 protrudes from the support sub-arm 210 (as shown in Figs. 3 and 5), thereby providing a space for mounting the threaded sleeve 520. The outer peripheral surface of the support sub-arm 210 refers to the outer surface of the support sub-arm 210 surrounding the axis of the positioning seat 100. In an alternative embodiment, the distance from the outer circumferential surface of the first positioning sub-seat 110 to the axis of the positioning seat 100 may be equal to the distance from the outer circumferential surface of the support sub-arm 210 to the axis of the positioning seat 100 (as shown in Fig. 9). It can be properly designed in practice according to the shape, size and assembly of the housing 30 and other components.

In addition, the embodiment of the present invention also provides a positioning structure that is implemented as the aforementioned positioning structure 20.

The embodiment of the present invention also provides a medical device. As shown in Fig. 10, the medical device includes a catheter assembly 60 and the aforementioned handle. The catheter assembly 60 includes a movable tube, and the proximal end of the movable tube is connected to the distal end of the moving mechanism 40. When the moving mechanism 40 moves along the axial direction of the positioning structure, the movable tube is driven to move along the axial direction of the positioning structure.

Specifically, the catheter assembly 60 includes an inner tube 610 and an outer tube 620 sleeved outside the inner tube 610 (as annotated in Fig. 2). Either the inner tube 610 or the outer tube 620 is used as the movable tube. When the outer tube 620 is used as the movable tube, the moving mechanism 40 further includes a transition tube 430. The distal end of the transition tube 430 is connected to the proximal end of the outer tube. The proximal end of the transition tube 430 is connected to the distal end of the moving rod 410. Moreover, the moving rod 410 also has a second inner cavity 440 extending axially through the moving rod 410, and the second inner cavity 440 is connected with the outer tube 620. The proximal end of the inner tube passes through the proximal end of the outer tube and also passes through the second inner cavity 440. When the first inner cavity 101 extends through the second positioning sub-seat 120 in the first direction, the proximal end of the inner tube further passes through the second positioning sub-seat 120 along the first inner cavity 101 to connect with other mechanisms that are provided according to actual conditions. Alternatively, when the proximal end of the first inner cavity 101 is a closed end, the proximal end of the inner tube may be fixedly connected to the proximal wall of the first inner cavity 101.

The use of the medical device will be introduced below by using the outer tube 620 as the movable tube.

The medical implant is firstly loaded into the catheter assembly 60. Specifically, the medical implant is sleeved on the inner tube 610 and compressed by the outer tube 620.

The medical implant is then conveyed via the medical device to the target location in the patient's body.

Next, by operating the driving part 50 on the handle, the outer tube 620 is driven to move in the direction from distal to proximal to release the medical implant.

After that, the driving part 50 is operated again to drive the outer tube 620 to move in the direction from proximal to distal, so that the outer tube 620 and the inner tube 610 are closed (at this time, the distal end of the inner tube 610 returns back into the outer tube 620).

Finally, the entire medical device is removed out of the patient's body.

Although the present invention is disclosed above, it is not limited thereto. Various changes and modifications can be made to the present invention by those skilled in the art without departing from the scope of the invention. Accordingly, the invention is intended to embrace all such modifications and variations if they fall within the scope of the appended claims and equivalents thereof.

## Claims

1. A handle for interventional surgery, comprising:
a housing (30);
a positioning structure (10), at least partially disposed inside the housing (30), the positioning structure (10) having a first inner cavity (101), the positioning structure (10) comprising a positioning seat (100) and a support arm (200), the positioning seat (100) comprises a first positioning sub-seat (110) and a second positioning sub-seat (120), and wherein a distal end of the support arm (200) is connected to the first positioning sub-seat (110), and a proximal end of the support arm (200) is connected to the second positioning sub-seat (120), the positioning seat (100) connected to the housing (30), the support arm (200) being part of a side wall of the first inner cavity (101), and the support arm (200) extending along an axial direction of the first inner cavity (101);
a driving part (50) connected to the housing (30); and
a moving mechanism (40), comprises a moving rod (410) and a transmission part (420) provided on the moving rod (410), and the moving rod (410) is at least partially inserted in the first inner cavity (101), and wherein
the support arm (200) comprises at least two support sub-arms (210) arranged at intervals along a circumference of the positioning seat (100), and an avoidance slot (201) extending axially is formed between adjacent two of the support sub-arms (210) and is connected to the first inner cavity (101);
the transmission part (420) passes through the avoidance slot (201) and is drivingly connected with the driving part (50), the driving part (50) is configured for transmitting driving force through the transmission part (420), thereby driving the moving mechanism (40) to move axially relative to the positioning structure (10).

2. The handle for interventional surgery according to claim 1, wherein the number of avoidance slots (201) is the same as the number of support sub-arms (210)

3. The handle for interventional surgery according to claim 1, wherein the at least two support sub-arms (210) are arranged to be centrally or axially symmetrical along the circumference of the positioning seat (100); or
wherein the driving part (50) is provided with first transmission threads (521); a part of an outer surface of the moving rod (410) is provided with second transmission threads that is formed as the transmission part (420) and engaged with the first transmission threads (521).

4. The handle for interventional surgery according to claim 3, wherein the housing (30) comprises a main housing (310) and a cover (320), and wherein a distal end of the driving part (50) and a proximal end of the main housing (310) are rotatably connected, and a proximal end of the driving part (50) is rotatably connected to the cover (320).

5. The handle for interventional surgery according to claim 4, wherein the first positioning sub-seat (110) is connected to the main housing (310), and the second positioning sub-seat (120) is connected to the cover (320).

6. The handle for interventional surgery according to claim 5, wherein, in a cross section of the first positioning sub-seat (110), the first inner cavity (101) comprises a central area (101a) and an avoidance area (101b), and wherein the central area (101a) is configured for allowing the moving rod (410) to pass through, and the avoidance area (101b) is connected with the central area (101a) and is configured for the transmission part (420) to pass through, each avoidance area (101b) is located between adjacent two of the support sub-arms (210), the avoidance area (101b) is arranged in correspondence with the avoidance slot (201).

7. The handle for interventional surgery according to claim 5, wherein, in a radial direction of the positioning structure (10), an outer surface of the first positioning sub-seat (110) protrudes from an outer surface of the support arm (200).

8. The handle for interventional surgery according to claim 5, wherein the first positioning sub-seat (110) is configured to remain circumferentially stationary relative to the main housing (310).

9. The handle for interventional surgery according to claim 5, wherein the cover (320) has a central through hole (321) therein; the second positioning sub-seat (120) comprises a first seat body (121) and a second seat body (122), and wherein the first seat body (121) is connected to the support arm (200), the second seat body (122) is connected to a proximal end of the first seat body (121), and a cross-sectional area of the second seat body (122) is smaller than an end face area of the proximal end of the first seat body (121); the second seat body (122) passes through the central through hole (321).

10. The handle for interventional surgery according to claim 5, wherein the second positioning sub-seat (120) is configured to remain circumferentially stationary relative to the cover (320).

11. The handle for interventional surgery according to claim 1, wherein a distal end of the first inner cavity (101) is a first open end, and a proximal end of the first inner cavity (101) is a second open end or a closed end.

12. The handle for interventional surgery according to claim 1, wherein a weight-reducing structure is formed on the positioning seat (100) and/or the support arm (200) to reduce a weight of the handle.

13. The handle for interventional surgery according to claim 3, wherein each avoidance slot (201) is configured for allowing a threaded segment of the second transmission threads to pass through.

14. A medical device, comprising a catheter assembly (60) and the handle for interventional surgery according to any one of claims 1 to 13, the catheter assembly (60) comprising a movable tube, a proximal end of the movable tube is connected to a distal end of the moving mechanism (40) of the handle, wherein the handle is configured to control the movable tube to move axially.

15. The medical device according to claim 14, wherein the moving mechanism (40) has a second inner cavity (440) extending through the moving mechanism (40) along an axial direction thereof; the catheter assembly (60) comprises an inner tube (610) and an outer tube (620) sleeved outside the inner tube (610), and the outer tube (620) is used as the movable tube, the outer tube (620) is connected to the distal end of the moving mechanism (40), a proximal end of the inner tube (610) extends out of the outer tube (620) and extends through the second inner cavity (440).

## Patentansprüche

1. Griff für interventionelle chirurgische Eingriffe, umfassend:
ein Gehäuse (30);
eine Positionierstruktur (10), die zumindest teilweise innerhalb des Gehäuses (30) angeordnet ist, wobei die Positionierstruktur (10) einen ersten inneren Hohlraum (101) aufweist, die Positionierstruktur (10) einen Positioniersitz (100) und einen Stützarm (200) umfasst, der Positioniersitz (100) einen ersten Positionierteilsitz (110) und einen zweiten Positionierteilsitz (120) umfasst und wobei ein distales Ende des Stützarms (200) mit dem ersten Positionierteilsitz (110) verbunden ist und ein proximales Ende des Stützarms (200) mit dem zweiten Positionierteilsitz (120) verbunden ist, der Positioniersitz (100) mit dem Gehäuse (30) verbunden ist, der Stützarm (200) Teil einer Seitenwand des ersten inneren Hohlraums (101) ist und der Stützarm (200) sich in axialrichtung des ersten inneren Hohlraums (101) erstreckt;
ein Antriebsteil (50), das mit dem Gehäuse (30) verbunden ist; und
einen Bewegungsmechanismus (40), der eine bewegliche Stange (410) und ein an der beweglichen Stange (410) vorgesehenes Getriebeteil (420) umfasst, wobei die bewegliche Stange (410) zumindest teilweise in den ersten inneren Hohlraum (101) eingeführt ist, und wobei
der Stützarm (200) mindestens zwei Stützteilarme (210) umfasst, die in Abständen entlang eines Umfangs des Positioniersitzes (100) angeordnet sind, und ein sich axial erstreckender Ausweichschlitz (201) zwischen zwei benachbarten Stützteilarmen (210) gebildet und mit dem ersten inneren Hohlraum (101) verbunden ist;
das Getriebeteil (420) durch den Ausweichschlitz (201) verläuft und mit dem Antriebsteil (50) antriebsmäßig verbunden ist, wobei das Antriebsteil (50) so konfiguriert ist, dass es eine Antriebskraft über das Getriebeteil (420) überträgt und dadurch den Bewegungsmechanismus (40) axial relativ zur Positionierstruktur (10) bewegt.

2. Griff für interventionelle chirurgische Eingriffe nach Anspruch 1, wobei die Anzahl von Ausweichschlitzen (201) der Anzahl von Stützarmen (210) entspricht.

3. Griff für interventionelle chirurgische Eingriffe nach Anspruch 1, wobei die mindestens zwei Stützarme (210) zentrisch oder axial symmetrisch entlang des Umfangs des Positioniersitzes (100) angeordnet sind; oder
wobei das Antriebsteil (50) mit ersten Gewindegängen (521) versehen ist; ein Teil der Außenfläche der beweglichen Stange (410) mit zweiten Gewindegängen versehen ist, die als Getriebeteil(420) ausgebildet sind und mit den ersten Gewindegängen (521) in Eingriff stehen.

4. Griff für interventionelle chirurgische Eingriffe nach Anspruch 3, wobei das Gehäuse (30) ein Hauptgehäuse (310) und eine Abdeckung (320) umfasst und wobei ein distales Ende des Antriebsteils (50) und ein proximales Ende des Hauptgehäuses (310) drehbar miteinander verbunden sind und ein proximales Ende des Antriebsteils (50) drehbar mit der Abdeckung (320) verbunden ist.

5. Griff für interventionelle chirurgische Eingriffe nach Anspruch 4, wobei der erste Positionierteilsitz (110) mit dem Hauptgehäuse (310) verbunden ist und der zweite Positionierteilsitz (120) mit der Abdeckung (320) verbunden ist.

6. Griff für interventionelle chirurgische Eingriffe nach Anspruch 5, wobei in einem Querschnitt des ersten Positionierteilsitzes (110) der erste innere Hohlraum (101) einen zentralen Bereich (101a) und einen Ausweichbereich (101b) umfasst und wobei der zentrale Bereich (101a) so konfiguriert ist, dass die bewegliche Stange (410) hindurchtreten kann, und der Ausweichbereich (101b) mit dem zentralen Bereich (101a) verbunden und so konfiguriert ist, dass das Getriebeteil (420) hindurchtreten kann, wobei sich jeder Ausweichbereich (101b) zwischen zwei benachbarten Stützteilarmen (210) befindet und der Ausweichbereich (101b) entsprechend dem Ausweichschlitz (201) angeordnet ist.

7. Griff für interventionelle chirurgische Eingriffe nach Anspruch 5, wobei in radialer Richtung der Positionierstruktur (10) eine Außenfläche des ersten Positionierteilsitzes (110) aus einer Außenfläche des Stützarms (200) herausragt.

8. Griff für interventionelle chirurgische Eingriffe nach Anspruch 5, wobei der erste Positionierteilsitz (110) so konfiguriert ist, dass er in Umfangsrichtung relativ zum Hauptgehäuse (310) stationär bleibt.

9. Griff für interventionelle chirurgische Eingriffe nach Anspruch 5, wobei die Abdeckung (320) eine zentrale Durchgangsbohrung (321) aufweist; der zweite Positionierteilsitz (120) einen ersten Sitzkörper (121) und einen zweiten Sitzkörper (122) umfasst und wobei der erste Sitzkörper (121) mit dem Stützarm (200) verbunden ist, der zweite Sitzkörper (122) mit einem proximalen Ende des ersten Sitzkörpers (121) verbunden ist und eine Querschnittsfläche des zweiten Sitzkörpers (122) kleiner ist als eine Stirnfläche des proximalen Endes des ersten Sitzkörpers (121); der zweite Sitzkörper (122) durch die zentrale Durchgangsbohrung (321) verläuft.

10. Griff für interventionelle chirurgische Eingriffe nach Anspruch 5, wobei der zweite Positionierteilsitz (120) so konfiguriert ist, dass er in Umfangsrichtung relativ zur Abdeckung (320) stationär bleibt.

11. Griff für interventionelle chirurgische Eingriffe nach Anspruch 1, wobei ein distales Ende des ersten inneren Hohlraums (101) ein erstes offenes Ende ist und ein proximales Ende des ersten inneren Hohlraums (101) ein zweites offenes Ende oder ein geschlossenes Ende ist.

12. Griff für interventionelle chirurgische Eingriffe nach Anspruch 1, wobei am Positioniersitz (100) und/oder am Stützarm (200) eine gewichtsreduzierende Struktur zur Gewichtsreduzierung des Griffs gebildet ist.

13. Griff für interventionelle chirurgische Eingriffe nach Anspruch 3, wobei jeder Ausweichschlitz (201) so konfiguriert ist, dass ein Gewindesegment der zweiten Gewindegänge hindurchtreten kann.

14. Medizinische Vorrichtung, umfassend eine Katheteranordnung (60) und den Griff für interventionelle chirurgische Eingriffe nach einem der Ansprüche 1 bis 13, wobei die Katheteranordnung (60) ein bewegliches Rohr umfasst, ein proximales Ende des beweglichen Rohrs mit dem distalen Ende des Bewegungsmechanismus (40) des Griffs verbunden ist, wobei der Griff so konfiguriert ist, dass er eine axiale Bewegung des beweglichen Rohrs steuert.

15. Medizinische Vorrichtung nach Anspruch 14, wobei der Bewegungsmechanismus (40) einen zweiten inneren Hohlraum (440) aufweist, der sich in axialrichtung durch den Bewegungsmechanismus (40) erstreckt; die Katheteranordnung (60) ein Innenrohr (610) und ein Außenrohr (620), das das Innenrohr (610) ummantelt, umfasst, und das Außenrohr (620) als bewegliches Rohr dient, das Außenrohr (620) mit dem distalen Ende des Bewegungsmechanismus (40) verbunden ist, ein proximales Ende des Innenrohrs (610) aus dem Außenrohr (620) herausragt und sich durch den zweiten inneren Hohlraum (440) erstreckt.

## Revendications

1. Poignée pour la chirurgie interventionnelle, comprenant :
un logement (30) ;
une structure de positionnement (10), au moins partiellement disposée à l'intérieur du logement (30), la structure de positionnement (10) présentant une première cavité intérieure (101), la structure de positionnement (10) comprenant un siège de positionnement (100) et un bras de support (200), le siège de positionnement (100) comprenant un premier sous-siège de positionnement (110) et un deuxième sous-siège de positionnement (120), et dans laquelle une extrémité distale du bras de support (200) est connectée au premier sous-siège de positionnement (110), et une extrémité proximale du bras de support (200) est connectée au deuxième sous-siège de positionnement (120), le siège de positionnement (100) étant connecté au logement (30), le bras de support (200) faisant partie d'une paroi latérale de la première cavité intérieure (101), et le bras de support (200) s'étendant le long d'une direction axiale de la première cavité intérieure (101) ;
une partie motrice (50) reliée au logement (30) ; et
un mécanisme mobile (40), comprenant une tige mobile (410) et une partie de transmission (420) disposée sur la tige mobile (410), et la tige mobile (410) étant au moins partiellement insérée dans la première cavité intérieure (101), et dans laquelle
le bras de support (200) comprend au moins deux sous-bras de support (210) agencés selon des intervalles le long d'une circonférence du siège de positionnement (100), et une fente d'évitement (201) s'étendant axialement est formée entre deux des sous-bras de support adjacents (210) et est reliée à la première cavité intérieure (101) ;
la partie de transmission (420) passe à travers la fente d'évitement (201) et est reliée de manière motrice à la partie motrice (50), la partie motrice (50) est configurée pour transmettre la force motrice à travers la partie de transmission (420), entraînant ainsi le mécanisme mobile (40) à se déplacer axialement par rapport à la structure de positionnement (10).

2. Poignée pour chirurgie interventionnelle selon la revendication 1, dans laquelle le nombre de fentes d'évitement (201) est le même que le nombre de sous-bras de support (210)

3. Poignée pour chirurgie interventionnelle selon la revendication 1, dans laquelle les au moins deux sous-bras de support (210) sont agencés de manière à être symétriques centralement ou axialement le long de la circonférence du siège de positionnement (100) ; ou
dans laquelle la partie motrice (50) est munie d'un premier filetage de transmission (521) ; une partie d'une surface extérieure de la tige mobile (410) est munie d'un second filetage de transmission qui est formé comme la partie de transmission (420) et mis en prise avec le premier filetage de transmission (521).

4. Poignée pour chirurgie interventionnelle selon la revendication 3, dans laquelle le logement (30) comprend un logement principal (310) et un couvercle (320), et dans laquelle une extrémité distale de la partie motrice (50) et une extrémité proximale du logement principal (310) sont reliées de manière rotative, et une extrémité proximale de la partie motrice (50) est reliée de manière rotative au couvercle (320).

5. Poignée pour chirurgie interventionnelle selon la revendication 4, dans laquelle le premier sous-siège de positionnement (110) est relié au logement principal (310), et le deuxième sous-siège de positionnement (120) est relié au couvercle (320).

6. Poignée pour chirurgie interventionnelle selon la revendication 5, dans laquelle, dans une coupe transversale du premier sous-siège de positionnement (110), la première cavité intérieure (101) comprend une zone centrale (101a) et une zone d'évitement (101b), et dans laquelle la zone centrale (101a) est configurée pour permettre le passage de la tige mobile (410), et la zone d'évitement (101b) est reliée à la zone centrale (101a) et est configurée pour permettre le passage de la partie de transmission (420), chaque zone d'évitement (101b) est située entre deux sous-bras de support adjacents (210), la zone d'évitement (101b) est agencée au niveau de la fente d'évitement (201).

7. Poignée pour chirurgie interventionnelle selon la revendication 5, dans laquelle, dans une direction radiale de la structure de positionnement (10), une surface extérieure du premier sous-siège de positionnement (110) fait saillie d'une surface extérieure du bras de support (200).

8. Poignée pour chirurgie interventionnelle selon la revendication 5, dans laquelle le premier sous-siège de positionnement (110) est configuré pour rester circonférentiellement stationnaire par rapport au logement principal (310).

9. Poignée pour chirurgie interventionnelle selon la revendication 5, dans laquelle le couvercle (320) comporte un trou traversant central (321) ; le deuxième sous-siège de positionnement (120) comprend un premier corps de siège (121) et un deuxième corps de siège (122), et dans laquelle le premier corps de siège (121) est relié au bras de support (200), le deuxième corps de siège (122) est relié à une extrémité proximale du premier corps de siège (121), et une superficie en coupe transversale du deuxième corps de siège (122) est plus petite que la superficie de la face d'extrémité de l'extrémité proximale du premier corps de siège (121) ; le deuxième corps de siège (122) passe à travers le trou traversant central (321).

10. Poignée pour chirurgie interventionnelle selon la revendication 5, dans laquelle le deuxième sous-siège de positionnement (120) est configuré pour rester circonférentiellement stationnaire par rapport au couvercle (320).

11. Poignée pour chirurgie interventionnelle selon la revendication 1, dans laquelle une extrémité distale de la première cavité interne (101) est une première extrémité ouverte, et une extrémité proximale de la première cavité interne (101) est une deuxième extrémité ouverte ou une extrémité fermée.

12. Poignée pour chirurgie interventionnelle selon la revendication 1, dans laquelle une structure de réduction de poids est formée sur le siège de positionnement (100) et/ou le bras de support (200) pour réduire le poids de la poignée.

13. Poignée pour chirurgie interventionnelle selon la revendication 3, dans laquelle chaque fente d'évitement (201) est configurée pour permettre à un segment fileté des deuxièmes fils de transmission de passer à travers.

14. Dispositif médical, comprenant un ensemble cathéter (60) et la poignée pour chirurgie interventionnelle selon l'une quelconque des revendications 1 à 13, l'ensemble cathéter (60) comprenant un tube mobile, une extrémité proximale du tube mobile est connectée à une extrémité distale du mécanisme mobile (40) de la poignée, dans lequel la poignée est configurée pour commander le déplacement axial du tube mobile.

15. Dispositif médical selon la revendication 14, dans lequel le mécanisme mobile (40) comporte une deuxième cavité interne (440) traversant le mécanisme mobile (40) dans une direction axiale ; l'ensemble cathéter (60) comprend un tube interne (610) et un tube externe (620) gainé à l'extérieur du tube interne (610), le tube externe (620) servant de tube mobile, le tube externe (620) étant relié à l'extrémité distale du mécanisme mobile (40), une extrémité proximale du tube interne (610) s'étendant hors du tube externe (620) et traversant la seconde cavité interne (440).
